# FASCICULE DE BREVET EUROPEEN

(11) **EP 2 471 575 B1**
(45) Date de publication et mention de la délivrance du brevet: **21.11.2018**
(21) Numéro de dépôt: 11185520.1
(22) Date de dépôt: 17.10.2011
(51) Int. Cl.: A61N 1/368, A61N 1/365, A61N 1/362

(54) **Dispositif médical implantable actif à stimulation auriculaire pour le traitement de l'insuffisance cardiaque diastolique**
Implantierbare aktive medizinische Vorrichtung zur Aurikelstimulation für die Behandlung von diastolischer Herzinsuffizienz
Active implantable medical device with auricular stimulation for the treatment of diastolic cardiac insufficiency

(30) Priorité: 29.12.2010 FR 1061335
(43) Date de publication de la demande: 04.07.2012
(73) Titulaire: Sorin CRM SAS, 92140 Clamart Cedex (FR)
(72) Inventeur: Limousin, Marcel, 75014 Paris (FR)
(74) Mandataire: Grünecker Patent- und Rechtsanwälte PartG mbB

(56) Documents cités:
- WO-A2-2005/011803
- US-A1- 2002 151 934
- US-A1- 2007 179 541
- US-A1- 2007 179 542

## Description

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Elle concerne plus particulièrement les implants destinés à traiter l'insuffisance cardiaque, en alternative ou en complément au traitement des troubles du rythme cardiaque.

En effet, il a été proposé de traiter par stimulation contrôlée des cavités cardiaques certains troubles de contraction myocardique observés chez des patients en insuffisance cardiaque, ces troubles pouvant être spontanés ou induits par une stimulation traditionnelle. Cette thérapie a permis d'observer des résultats souvent spectaculaires pour des patients en insuffisance cardiaque non améliorée par les traitements classiques.

Cette thérapie vise à resynchroniser la contraction des cavités cardiaques (oreillette et ventricule, et les deux ventricules entre eux) de manière à améliorer l'état du patient par optimisation des différentes phases du cycle hémodynamique, ce cycle comprenant : pré-éjection, contraction isovolumique, éjection systolique, relaxation isovolumique, et enfin remplissage de la cavité.

La plupart de ces dispositifs mettent en oeuvre une technique dite "CRT" (*Cardiac Resynchronization Therapy*) ou "BVP" (*Bi-Ventricular Pacing*) consistant à délivrer en tant que de besoin des impulsions électriques permettant d'assurer une stimulation conjointe et permanente des deux ventricules, gauche et droit, afin de resynchroniser ces derniers. On implante à cet effet au patient un appareil muni d'électrodes permettant de stimuler l'un et l'autre ventricule, le dispositif appliquant entre les instants respectifs de stimulation des ventricules gauche et droit un délai dit "délai interventriculaire" (délai VV) ajusté de manière à resynchroniser la contraction des deux ventricules avec optimisation fine de l'état hémodynamique du patient. Un tel stimulateur CRT est par exemple divulgué dans le EP 1 108 446 A1 (ELA Médical).

Cette technique de resynchronisation biventriculaire ne s'adresse toutefois qu'à l'une des formes d'insuffisance cardiaque, dite "insuffisance systolique". Dans cette forme de la maladie, le muscle cardiaque est dans l'incapacité de fournir l'effort nécessaire pour assurer un débit cardiaque suffisant, et le patient présente des signes de dilatation entraînant un élargissement du complexe QRS, qui est l'expression d'un retard de dépolarisation du ventricule gauche, entraînant une désynchronisation. La stimulation biventriculaire CRT permet alors de resynchroniser les ventricules et de rendre plus homogène la contraction cardiaque.

L'autre forme d'insuffisance cardiaque, dite "insuffisance diastolique" ou "à fonction systolique préservée" ne présente pas de caractère de désynchronisation des ventricules, mais provient d'un défaut de remplissage du ventricule gauche.

Une stimulation CRT biventriculaire sera donc sans effet dans ce cas de figure.

Cette pathologie touche toutefois près de 40 % des insuffisants cardiaques, et il n'existe pas de traitement efficace connu pour y remédier.

Le point de départ de l'invention est la constatation clinique du fait que cette forme de pathologie peut notamment être la conséquence d'un trouble de conduction au niveau des oreillettes (bloc interauriculaire), qui induit un retard de la dépolarisation, et donc de la contraction, de l'oreillette gauche par rapport à l'oreillette droite. En revanche, comme les voies de conduction auriculo-ventriculaires se comportent par ailleurs normalement, la dépolarisation et la contraction des deux ventricules survient dans un délai normal, donc sans désynchronisation entre les ventricules. Dès lors, le bloc interauriculaire introduit une mauvaise synchronisation entre la contraction de l'oreillette gauche et du ventricule gauche : le retard de la contraction de l'oreillette gauche fait que celle-ci se contracte pratiquement en même temps que le ventricule gauche, et donc ne peut pas remplir correctement sa fonction, qui est de terminer le remplissage du ventricule gauche.

Le US 2005/0102002 A1 propose un dispositif pourvu d'un capteur mesurant un paramètre hémodynamique (capteur de pression intraventriculaire, capteur de débit veineux pulmonaire, capteur de débit au travers de la valve mitrale, capteur acoustique, accéléromètre), dont le signal est utilisé pour calculer un indice représentatif de la fonction diastolique du patient. Les paramètres de stimulation sont modifiés en tant que de besoin de manière à maximiser cet indice de performance, par exemple par une sélection appropriée des sites de stimulation et/ou un contrôle du délai auriculo-ventriculaire.

Le US 2007/0179542 A1 propose un autre dispositif du même type, comportant un capteur délivrant un signal d'accélération LV représentatif des contractions du ventricule gauche. Le dispositif comprend également des moyens pour faire varier les délais auriculo-ventriculaire (AV) et interauriculaire (AA) en fonction de divers paramètres, notamment le signal d'accélération LV du ventricule gauche, pour tenter d'améliorer la fonction cardiaque du patient. Ce document prévoit en particulier d'ajuster itérativement le délai AV en fonction du signal d'accélération LV mesuré, jusqu'à trouver une valeur optimale de séquencement entre les contractions de l'oreillette et celles du ventricule.

D'autres techniques ont été proposées pour traiter l'insuffisance cardiaque diastolique, consistant à opérer une stimulation prématurée de l'oreillette gauche :
- soit par "overdrive", technique consistant à stimuler l'oreillette gauche à une fréquence légèrement supérieure à la fréquence spontanée du rythme sinusal (donc du rythme de l'oreillette droite),
- soit en déclenchant la stimulation de l'oreillette gauche dès détection de la dépolarisation de l'oreillette droite.

Ces deux méthodes sont purement électriques (elles sont basées sur le recueil des signaux de dépolarisation), et leur efficacité ne peut être validée que par une analyse hémodynamique, par exemple par un examen échocardiographique. En outre, elles ne procurent aucune adaptation à l'effort en cas de changement d'activité du patient, ni en cas d'évolution éventuelle de la conduction du tissu cardiaque sur le long terme.

L'un des buts de l'invention est de proposer une nouvelle technique de traitement de l'insuffisance cardiaque diastolique par bloc interauriculaire, qui pallie les inconvénients des méthodes proposées jusqu'à présent.

Un autre but de l'invention est de proposer une telle technique qui permette de s'affranchir de la mesure et du calcul d'un indice de performance diastolique, de manière à pouvoir assurer un rétablissement de la fonction diastolique de manière à la fois simple (en termes de moyens de calcul) et très réactive (efficacité obtenue cycle à cycle).

L'invention propose à cet effet d'opérer par stimulation auriculaire exclusive (stimulation biauriculaire), avec une stimulation coordonnée des deux oreillettes permettant de rétablir un séquencement satisfaisant de la contraction de l'oreillette gauche par rapport à celle du ventricule, de manière que l'oreillette puisse remplir correctement sa fonction d'achèvement du remplissage du ventricule gauche.

Pour mette en oeuvre l'invention, il n'est pas besoin de disposer de moyens de stimulation ventriculaire et/ou de recueil des dépolarisations ventriculaires. Ces derniers peuvent certes exister, par exemple dans le cas d'un stimulateur multisite assurant, outre la stimulation des oreillettes, celle du ventricule droit ou même celle des deux ventricules (stimulateur quadruple chambre). Mais ces moyens ventriculaires ne sont pas impliqués dans le traitement de l'insuffisance cardiaque diastolique, qui vise à pallier un trouble préexistant de conduction entre les oreillettes, à savoir un bloc interauriculaire se traduisant par un retard excessif (voire même une absence) de conduction entre l'oreillette droite et l'oreillette gauche. Essentiellement, l'invention propose d'utiliser un capteur délivrant un signal représentatif des mouvements mécaniques produits par les contractions cycliques du coeur, par exemple un capteur de pression ou, avantageusement, un capteur d'accélération endocardiaque (EA) incorporé à une sonde implantée, comme décrit par exemple dans le EP 0 515 319 A1 (Sorin Biomedica Cardio SpA). Ce document enseigne la manière de recueillir un signal EA au moyen d'une sonde endocavitaire pourvue d'une électrode distale de stimulation implantée au fond du ventricule et intégrant un microaccéléromètre couplé au muscle cardiaque et permettant de mesurer l'accélération endocardiaque.

On notera toutefois que, bien que dans la présente description on fasse principalement référence à l'analyse d'un signal EA délivré par un capteur placé sur une sonde endocavitaire, l'invention est également applicable à une analyse opérée à partir d'un signal EA délivré par d'autres types de capteurs implantés, tels qu'un capteur de mouvement d'une paroi du myocarde, un capteur épicardique ou un accéléromètre placé dans le boîtier d'un implant, dès lors qu'il permet de recueillir un signal d'accélération endocardiaque (signal EA) représentatif des contractions de l'oreillette.

On notera qu'un tel capteur délivre un signal fonctionnel (le signal EA) représentatif de la mécanique cardiaque, et non un signal issu de la propagation électrique de l'onde de dépolarisation comme celui délivré à partir des électrodes de recueil de la sonde.

Diverses études cliniques ont montré que l'accélération endocardiaque est un paramètre qui reflète très précisément et en temps réel, aussi bien dans le cas d'un fonctionnement normal que dans celui d'un fonctionnement déficient, les phénomènes mécaniques liés aux mouvements des cavités cardiaques - notamment des oreillettes, dans le cas de la présente invention.

En particulier, la contraction auriculaire (systole) s'exprime par la présence dans le signal EA d'une composante spécifique, dite EA4, qu'il est possible de distinguer de la contraction des ventricules. D'autre part, la masse cardiaque la plus importante étant dans les cavités gauches, la composante EA4 du signal EA correspond essentiellement à l'activité de l'oreillette gauche.

Un des effets de la systole auriculaire est de terminer la mise sous tension du muscle ventriculaire et de provoquer le début de la contraction du ventricule gauche. Mais dans le cas d'un patient souffrant d'insuffisance cardiaque diastolique consécutive à un bloc interauriculaire, le retard de la contraction de l'oreillette gauche fera en sorte que la composante EA4 associée à la contraction auriculaire (principalement, à la contraction de l'oreillette gauche) se trouvera noyée dans la contraction ventriculaire, et ne pourra pas être détectée et isolée sur le signal EA recueilli.

Pour retrouver une systole auriculaire gauche effective, discernable de la systole ventriculaire sur le signal EA4, l'invention propose, essentiellement, de stimuler l'oreillette gauche avec un délai interauriculaire progressivement réduit, jusqu'à retrouver sur le signal EA une contraction auriculaire présentant une prématurité suffisante pour la distinguer de la contraction du ventricule gauche. Cette configuration correspond à un séquencement correct de la contraction des deux cavités gauches, et donc à une fonction diastolique conforme.

Plus précisément, l'invention propose un dispositif comprenant, de manière en elle-même connue et divulguée par le US 2007/0179542 A1 précité : des moyens de recueil de dépolarisations auriculaires droites ; des moyens de recueil de dépolarisations auriculaires gauches ; des moyens de délivrance d'impulsions de stimulation auriculaire gauche ; un capteur de contractions, apte à délivrer un signal de contraction représentatif des mouvements produits par les contractions cycliques du myocarde ; des moyens d'analyse du signal de contraction, aptes à révéler la survenue d'une contraction auriculaire gauche effective, temporellement discernable de la contraction ventriculaire du même cycle cardiaque ; et des moyens de contrôle, aptes à déterminer et appliquer aux moyens de délivrance d'impulsions de stimulation auriculaire gauche un délai interauriculaire entre le recueil d'une dépolarisation auriculaire droite et la délivrance d'une impulsion de stimulation auriculaire gauche, ce délai interauriculaire étant modifiable en fonction du signal de contraction.

De façon caractéristique, les moyens de contrôle sont des moyens aptes : en cas d'absence de contraction auriculaire gauche révélée par les moyens d'analyse du signal de contraction, à réduire itérativement le délai interauriculaire au cours de cycles cardiaques successifs, depuis une valeur initiale jusqu'à une valeur d'ajustement assurant la survenue d'une contraction auriculaire gauche révélée par les moyens d'analyse du signal de contraction ; et à maintenir ensuite le délai interauriculaire à cette valeur d'ajustement tant que la présence d'une contraction auriculaire gauche est avérée par les moyens d'analyse du signal de contraction, de manière à restaurer la fonction diastolique du patient.

Selon diverses caractéristiques subsidiaires avantageuses :
- le capteur de contractions est disposé sur une sonde apte à être implantée au niveau de l'une des oreillettes, notamment l'oreillette droite ;
- le capteur de contractions est un capteur d'accélération endocardiaque ;
- les moyens d'analyse comprennent alors des moyens aptes à reconnaître et isoler dans le signal délivré par le capteur une composante EA4 correspondant au quatrième pic d'accélération endocardiaque associé à l'activité auriculaire, la présence de cette composante EA4 révélant la survenue d'une contraction auriculaire gauche temporellement discernable de la contraction ventriculaire du même cycle cardiaque ;
- en variante, le capteur de contractions peut être un capteur de pression cardiaque endocavitaire gauche ;
- le dispositif comprend en outre des moyens pour mesurer, sur un rythme spontané en l'absence de stimulation auriculaire, l'intervalle de temps séparant le recueil d'une dépolarisation auriculaire droite et celui d'une dépolarisation auriculaire gauche consécutive, et pour déterminer ladite valeur initiale de délai interauriculaire à partir de l'intervalle ainsi mesuré ;
- le dispositif comprend en outre des moyens aptes, au cas où la valeur d'ajustement est abaissée au-dessous d'une valeur minimale donnée sans que la présence d'une contraction auriculaire gauche soit détectée par les moyens d'analyse, à commander la délivrance des impulsions de stimulation auriculaire gauche avec un intervalle de couplage itérativement réduit jusqu'à une valeur assurant l'apparition de ladite contraction auriculaire gauche, l'intervalle de couplage étant ensuite maintenu à cette valeur tant que la présence d'une contraction auriculaire gauche est avérée par les moyens d'analyse ;
- le dispositif est essentiellement dépourvu de moyens de délivrance d'impulsions de stimulation ventriculaires, et de moyens de recueil de dépolarisations ventriculaires.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 illustre un exemple de signal d'accélération endocardiaque EA recueilli au cours de trois cycles cardiaques successifs.
La Figure 2 est une série de trois chronogrammes illustrant différents signaux caractérisant l'activité cardiaque au cours d'un cycle donné.
La Figure 3 illustre de façon schématique la position des différents sites impliqués dans l'activité électrique cyclique, spontanée ou stimulée, du coeur.
La Figure 4 est un organigramme des différentes étapes mises en oeuvre par l'invention.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Ovatio* et *Paradym* produits et commercialisés par Sorin CRM, Clamart, France.

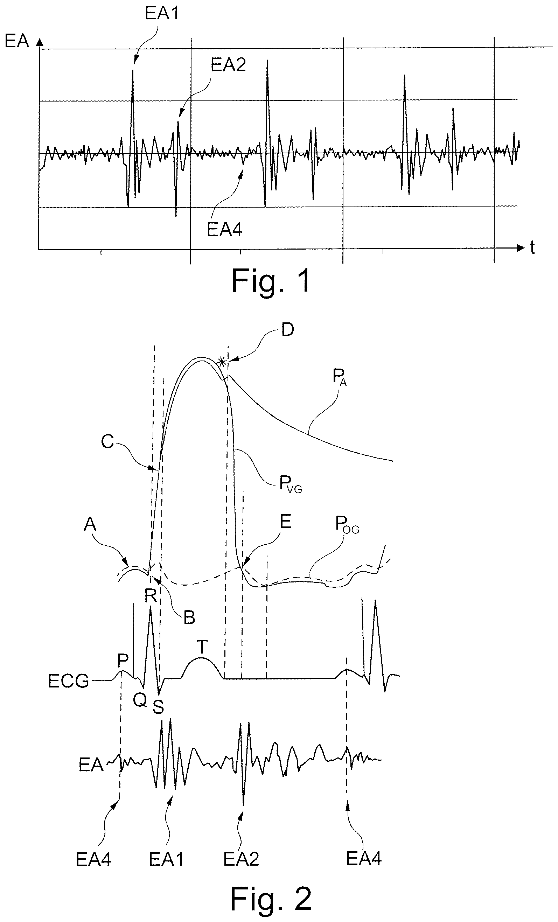

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail. Comme illustré sur la Figure 1, le signal d'accélération endocardiaque EA recueilli au cours d'un cycle cardiaque donné (ci-après "signal EA") forme deux composantes principales (ci-après "composantes EA"), correspondant aux deux bruits majeurs du coeur (sons S1 et S2 du phonocardiogramme) qu'il est possible de reconnaître dans chaque cycle cardiaque :
- la première composante d'accélération endocardiaque ("composante EA1"), dont les variations d'amplitude sont étroitement liées aux variations de la pression dans le ventricule (l'amplitude maximale crête-à-crête, appelée "PEA1 ", de cette composante EA1 étant plus précisément corrélée au maximum positif de la variation de pression dP/dt dans le ventricule gauche) et peuvent donc constituer un paramètre représentatif de la contractilité du myocarde, elle-même liée au niveau d'activité du système sympathique ;
- la seconde composante d'accélération endocardiaque ("composante EA2") qui, quant à elle, survient pendant la phase de relaxation ventriculaire isovolumique et de décélération brusque de la masse sanguine en mouvement dans l'aorte. Cette seconde composante est principalement produite par la fermeture des valves aortiques et pulmonaires.

Le signal EA contient également deux autres composantes, d'amplitude très inférieure, appelées EA3 et EA4, correspondant aux sons S3 et S4 du phonocardiogramme.

L'invention s'intéresse plus particulièrement à la composante EA4, qui est directement liée à la présence d'une contraction auriculaire.

Comme on peut le voir sur la Figure 1, cette composante se situe immédiatement avant la composante EA1. Pour cette raison, elle est quelquefois désignée "EA0" par les rythmologues, dans la mesure où, du point de vue électrique, la contraction de l'oreillette précède la contraction du ventricule ; en revanche, si l'on se place du point de vue du flux sanguin pompé par le myocarde, la contraction de l'oreillette (correspondant à la composante EA4) achève le remplissage du ventricule en fin de diastole (composante EA2) et se situe donc, du point de vue de l'hémodynamique cardiaque, après cette dernière - d'où la désignation "EA4".

La présence de cette composante EA4 peut être recherchée par une technique d'analyse du signal EA telle que celle décrite par exemple dans les EP 2 189 180 A1 ou EP 2 189 182 A1 (ELA Medical), permettant notamment de rechercher et confirmer la présence d'une composante EA4, et définir avec précision les instants de début et de fin de celle-ci dans le cycle cardiaque.

La Figure 2 illustre les différents signaux caractérisant l'activité du coeur au cours d'un cycle cardiaque, avec : le profil des pressions intracardiaques, un relevé d'électrocardiogramme de surface (ECG), et les variations du signal d'accélération endocardiaque (EA).

Sur le profil des pressions intracardiaques, la caractéristique P_{A} illustre les variations de la pression aortique, P_{VG} celles du ventricule gauche et P_{OG} celles dans l'oreillette gauche. Les points A à E correspondent aux différentes phases suivantes : A, contraction de l'oreillette gauche ; B, fermeture de la valvule mitrale ; C, ouverture de la valvule aortique ; D, fermeture de la valvule aortique ; et E, ouverture de la valvule mitrale.

Le signal ECG comprend successivement : l'onde P correspondant à la dépolarisation de l'oreillette, le complexe QRS correspondant à la dépolarisation du ventricule et l'onde T de repolarisation ventriculaire.

Le signal d'accélération endocardiaque EA, quant à lui, peut se décomposer de la manière suivante : la composante EA4 correspond à la contraction de l'oreillette (onde P), et elle est suivie par la composante EA1, qui débute à la suite du complexe QRS et est engendrée par une combinaison de la fermeture des valves auriculo-ventriculaires, de l'ouverture des valves semi-lunaires et de la contraction du ventricule gauche. La composante EA2 qui lui fait suite accompagne la fin de la systole ventriculaire et elle est générée par la fermeture des valves semi-lunaires.

Le signal EA est donc un signal fonctionnel représentatif de la mécanique cardiaque, distinct du signal électrique de dépolarisation recueilli après survenue d'un événement spontané ou stimulé - dans le cas présent, un événement auriculaire, droit ou gauche.

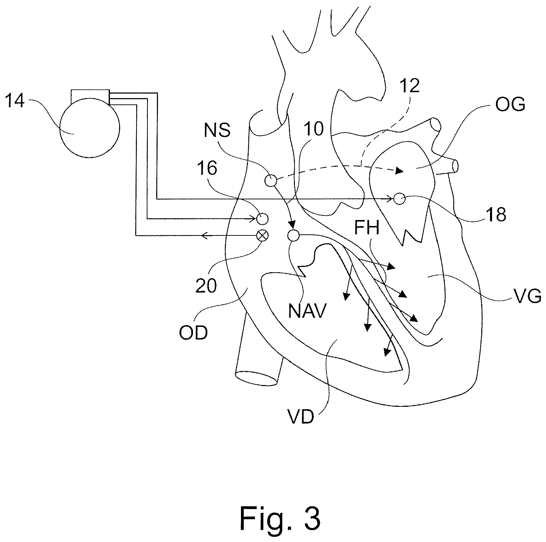

La Figure 3 illustre de façon schématique le coeur avec ses quatre cavités : oreillette droite OD, ventricule droit VD, oreillette gauche OG et ventricule gauche VG.

La contraction coordonnée des différentes cavités prend sa source au noeud sinusal NS, puis l'onde de dépolarisation est conduite au noeud atrio-ventriculaire NAV (conduction schématisée par la flèche 10), puis à partir de ce noeud au faisceau de His FH, et enfin aux tissus des ventricules droit et gauche VD et VG, provoquant la contraction de ceux-ci.

Par ailleurs, l'onde de dépolarisation issue du noeud sinusal NS provoque la contraction de l'oreillette droite OD puis, après conduction interauriculaire (conduction schématisée par la flèche 12) jusqu'à l'oreillette gauche OG, provoque la contraction de cette dernière.

Dans le cas d'un patient souffrant d'insuffisance cardiaque diastolique, la conduction auriculo-ventriculaire (flèche 10, du noeud sinusal NS au noeud atrio-ventriculaire NAV) est préservée, de même que les voies de conduction permettant d'assurer une contraction synchrone des deux ventricules droite et gauche VD et VG.

En revanche, la conduction interauriculaire (flèche 12) peut être altérée, entraînant un retard de la dépolarisation et donc de la contraction de l'oreillette gauche OG par rapport aux ventricules. Ceci induit un mauvais séquencement de la contraction de l'oreillette gauche OG par rapport à celle du ventricule gauche VG, avec une contraction plus ou moins concomitante de ces deux cavités. De ce fait, l'oreillette gauche OG ne peut plus remplir correctement sa fonction, qui est de terminer le remplissage du ventricule gauche VG.

L'invention propose une technique de diagnostic et de thérapie de cette pathologie, au moyen d'un stimulateur agissant uniquement sur les deux oreillettes.

Ce stimulateur comporte un générateur 14 relié à une électrode 16 de stimulation/recueil des dépolarisations au niveau de l'oreillette droite OD, et à une électrode 18 de stimulation/recueil des dépolarisations au niveau de l'oreillette gauche OG. L'électrode 18 peut être notamment positionnée dans le sinus coronaire, ou bien dans le septum interauriculaire, ou encore directement dans l'oreillette gauche après ponction du septum interauriculaire.

La sonde placée au niveau de l'oreillette droite est par ailleurs équipée d'un capteur 20 apte à délivrer un signal fonctionnel représentatif des contractions (et non des dépolarisations) des cavités cardiaques, typiquement un signal d'accélération endocardiaque EA du type exposé plus haut en référence aux Figures 1 et 2. L'électrode 16 et le capteur 20 sont disposés sur une sonde endocavitaire conventionnelle, implantée au niveau de l'oreillette droite via le réseau veineux.

Le capteur 20 sera sensible aux accélérations des parois cardiaques, avec une amplitude de signal correspondant principalement à l'activité de l'oreillette gauche et du ventricule gauche, dans la mesure où la masse cardiaque la plus importante est celle des cavités gauches (et ceci bien que le capteur soit implanté côté droit).

Dans l'exemple illustré, le capteur de contractions 20 est disposé au niveau des oreillettes, sur une sonde auriculaire, car le dispositif est du type "double chambre auriculaire", dépourvu de moyens de détection/stimulation ventriculaire. Il est en outre disposé sur la sonde auriculaire droite car celle-ci est d'un type qui se prête plus facilement à l'intégration d'un capteur (sonde endocavitaire) que dans le cas d'une sonde auriculaire gauche (sonde très fine, introduite dans le réseau coronaire ou bien au travers du septum interauriculaire). Mais d'autres placements du capteur d'accélération sont envisageables, par exemple sur une sonde ventriculaire dans le cas d'un stimulateur de type "triple chambre/double auriculaire", dès lors que le signal recueilli permet de caractériser la contraction de l'oreillette (composante EA4 suffisamment discernable sur le signal recueilli).

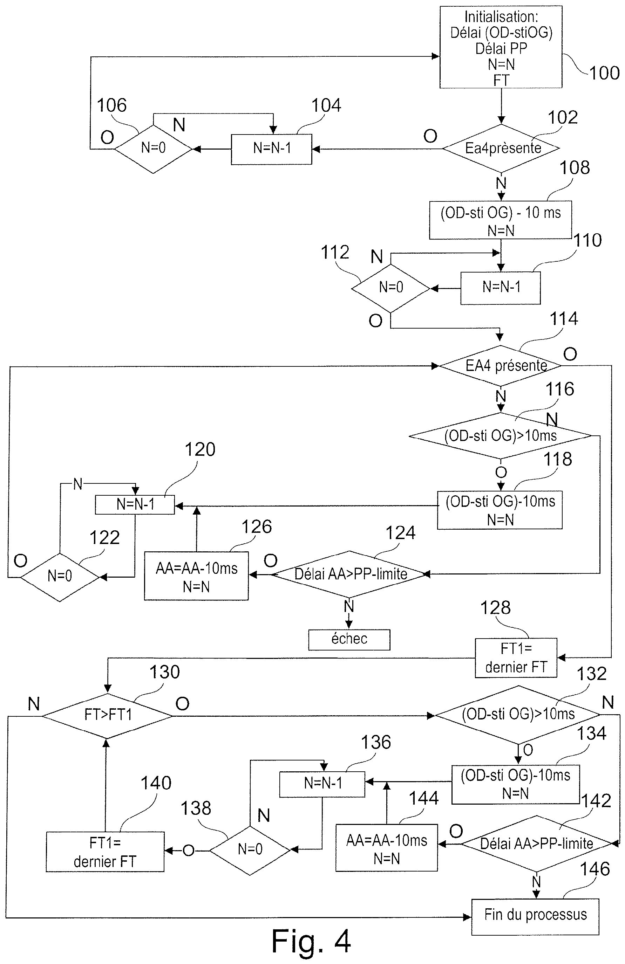

La Figure 4 est un organigramme détaillé montrant la manière dont est opéré le traitement des signaux recueillis par les électrodes 16, 18 et le capteur de contractions 20, pour la mise en oeuvre de l'invention.

La première phase (étape 100) consiste à opérer une analyse initiale en rythme spontané avec : mesure du délai interauriculaire (désigné "OD-sti OG") séparant les dépolarisations spontanées de l'oreillette droite et de l'oreillette gauche, mesure de l'intervalle de couplage auriculaire PP (délai séparant deux dépolarisations auriculaires de la même cavité, représentatif de la durée d'un cycle en rythme spontané), et mesure du temps de remplissage FT. Le compteur de cycles par ailleurs initialisé a une valeur prédéterminée N.

Le capteur de contractions 20 analyse ensuite le signal EA pour déterminer si une composante EA4 est présente ou non (étape 102). L'absence de composante EA4 détectable signifie que la contraction de l'oreillette gauche OG est noyée dans celle de la contraction du ventricule gauche VG, et donc que l'oreillette se contracte tardivement et ne remplit pas sa fonction d'achèvement du remplissage du ventricule gauche en fin de diastole de ce dernier. Une composante EA4 détectable signifie que la contraction de l'oreillette gauche OG intervient avant la contraction du ventricule gauche, donc en fin de diastole, avec un séquencement correct permettant à la contraction de l'oreillette d'achever le remplissage du ventricule avant que ce dernier ne commence à se contracter.

Si la composante EA4 est présente, il n'y a pas lieu de prendre d'action particulière, et le processus attend l'écoulement des N cycles (étapes 104, 106) avant de réitérer la phase précédente d'initialisation de l'étape 100. Si à l'étape 102 l'absence de composante EA4 est confirmée, après détection de la contraction spontanée de l'oreillette droite OD une stimulation est délivrée à l'oreillette gauche OG, avec application d'un retard (désigné "OD-sti OG") correspondant au délai interauriculaire spontané mesuré à l'étape 100, diminué d'un pas fixe, par exemple un pas de 10 ms (étape 108). Le compteur de cycles est par ailleurs réinitialisé à N.

Après écoulement de N cycles (étapes 110, 112), la présence d'une composante EA4 est à nouveau testée (étape 114).

Si la composante EA4 est présente, la stimulation est maintenue avec le même retard (étapes 128 à 146, décrites plus bas).

Si la composante EA4 est absente, et si le délai de stimulation auriculaire OD-sti OG n'est pas à son minimum (étape 116), alors ce délai est encore réduit d'un pas, et la stimulation est délivrée dans ces conditions pendant N cycles (étapes 118, 120 et 122, homologues des étapes 108, 110 et 112 ci-dessus).

Si, à l'étape 116, (i) le délai de stimulation auriculaire est réduit au minimum (10 ms ou moins), (ii) la composante EA4 est toujours absente, et (iii) l'intervalle de stimulation auriculaire AA est supérieur à l'intervalle de couplage PP en rythme spontané, à une valeur limite près, alors une stimulation auriculaire gauche est délivrée avec un intervalle AA égal à la durée du cycle sinusal diminué de 10 ms (étape 126), et ainsi de suite pendant N cycles (étapes 120, 122) jusqu'à apparition d'une composante EA4 (étape 114 ci-dessus).

Avantageusement, mais de manière facultative, une fois que la composante EA4 a été détectée au test de l'étape 114, le processus réalise une optimisation supplémentaire par mesure comparative du temps de remplissage FT.

Ces aspects sont exposés notamment dans la demande EP 2 206 531 A1 (ELA Medical), qui décrit la manière d'analyser un certain nombre de paramètres hémodynamiques, dont le temps de remplissage FT, pour optimiser l'ajustement des paramètres de stimulation.

Le temps de remplissage FT est l'intervalle de temps séparant la fermeture de la valvule aortique de la fermeture de la valvule mitrale ; il est généralement exprimé de manière relative, en pourcentage de la durée complète d'un cycle (durée RR), avec une valeur idéale typiquement FT > 40 %. Les instants des différentes phases hémodynamiques d'un même cycle, permettant en particulier de déterminer le temps de remplissage FT, peuvent notamment être déterminés par une technique telle que celle décrite dans le EP 2 092 885 A1 (ELA Médical), où les différents marqueurs temporels des instants caractéristiques du cycle cardiaque sont déterminés par l'analyse d'un signal EA. Les données fournies par le signal EA reflètent en effet très précisément et en temps réel, comme on l'a expliqué plus haut, les phénomènes concourant au fonctionnement mécanique du coeur et permettent ainsi, après filtrage et analyse, de fournir des marqueurs temporels de la systole et d'autres indices de performance hémodynamique du myocarde. Ces paramètres peuvent être déterminés en temps réel, battement après battement, ce qui permet d'optimiser sans délai la thérapie appliquée au patient.

Dans le cas présent, une mesure comparative est effectuée entre le temps de remplissage en rythme spontané (paramètre FT mesuré à l'étape initiale 100) et celui mesuré après réapparition de la composante EA4 suite à une stimulation contrôlée.

Ainsi, après avoir mesuré la valeur FT1 du dernier temps de remplissage (étape 128), on examine si ce temps de remplissage a augmenté par rapport à la valeur initiale mesurée à l'étape 100, en rythme spontané (étape 130) :
- si le temps de remplissage a augmenté :
   - si le délai de stimulation auriculaire OD-sti OG est supérieur à son minimum, 10 ms (étape 132), alors ce délai est diminué (étape 134) et la stimulation est poursuivie sur ces bases pendant N cycles (étapes 136, 138 et 140), jusqu'à exécuter à nouveau le test de l'étape 130 ;
   - si la stimulation est en "overdrive" auriculaire (étape 142, identique à l'étape 124), alors l'intervalle de stimulation est diminué de 10 ms (étape 144, identique à l'étape 126), et comme précédemment la stimulation est poursuivie sur ces paramètres sur N cycles (étapes 136, 138 et 140) ;
- si après cette réduction le temps de remplissage augmente (test à nouveau de l'étape 130), alors on continue de réduire le délai de stimulation auriculaire, jusqu'à obtenir un temps de remplissage maximal (réitération des étapes 134 à 140) ; dans le cas contraire, les délais sont reprogrammés à la valeur précédente (fin du processus, étape 146).

## Revendications

1. Un dispositif médical implantable actif du type prothèse cardiaque de stimulation, resynchronisation et/ou défibrillation, comprenant :
- des moyens (16) de recueil de dépolarisations auriculaires droites ;
- des moyens (18) de recueil de dépolarisations auriculaires gauches ;
- des moyens (18) de délivrance d'impulsions de stimulation auriculaire gauche ;
- un capteur de contractions (20), apte à délivrer un signal de contraction représentatif des mouvements produits par les contractions cycliques du myocarde ;
- des moyens d'analyse du signal de contraction, aptes à révéler la survenue d'une contraction auriculaire gauche effective, temporellement discernable de la contraction ventriculaire du même cycle cardiaque ;
- des moyens de contrôle, aptes à déterminer et appliquer aux moyens de délivrance d'impulsions de stimulation auriculaire gauche un délai interauriculaire entre le recueil d'une dépolarisation auriculaire droite et la délivrance d'une impulsion de stimulation auriculaire gauche, ce délai interauriculaire étant modifiable en fonction du signal de contraction,
**caractérisé en ce que** les moyens de contrôle sont des moyens aptes :
• en cas d'absence de contraction auriculaire gauche révélée par les moyens d'analyse du signal de contraction, à réduire itérativement le délai interauriculaire au cours de cycles cardiaques successifs, depuis une valeur initiale jusqu'à une valeur d'ajustement assurant la survenue d'une contraction auriculaire gauche révélée par les moyens d'analyse du signal de contraction, et
• à maintenir ensuite le délai interauriculaire à cette valeur d'ajustement tant que la présence d'une contraction auriculaire gauche est avérée par les moyens d'analyse du signal de contraction,
de manière à restaurer la fonction diastolique du patient.

2. Le dispositif de la revendication 1, dans lequel le capteur de contractions est disposé sur une sonde apte à être implantée au niveau de l'une des oreillettes.

3. Le dispositif de la revendication 1, dans lequel le capteur de contractions est disposé sur une sonde apte à être implantée au niveau de l'oreillette droite.

4. Le dispositif de la revendication 1, dans lequel le capteur de contractions est un capteur d'accélération endocardiaque.

5. Le dispositif de la revendication 4, dans lequel les moyens d'analyse comprennent des moyens aptes à reconnaître et isoler dans le signal délivré par le capteur une composante EA4 correspondant au quatrième pic d'accélération endocardiaque associé à l'activité auriculaire, la présence de cette composante EA4 révélant la survenue d'une contraction auriculaire gauche temporellement discernable de la contraction ventriculaire du même cycle cardiaque.

6. Le dispositif de la revendication 1, dans lequel le capteur de contractions est un capteur de pression cardiaque endocavitaire gauche.

7. Le dispositif de la revendication 1, comprenant en outre des moyens pour mesurer, sur un rythme spontané en l'absence de stimulation auriculaire, l'intervalle de temps séparant le recueil d'une dépolarisation auriculaire droite et celui d'une dépolarisation auriculaire gauche consécutive, et pour déterminer ladite valeur initiale de délai interauriculaire à partir de l'intervalle ainsi mesuré.

8. Le dispositif de la revendication 1, comprenant en outre des moyens aptes, au cas où la valeur d'ajustement est abaissée au-dessous d'une valeur minimale donnée sans que la présence d'une contraction auriculaire gauche soit détectée par les moyens d'analyse, à commander la délivrance des impulsions de stimulation auriculaire gauche avec un intervalle de couplage itérativement réduit jusqu'à une valeur assurant l'apparition de ladite contraction auriculaire gauche, l'intervalle de couplage étant ensuite maintenu à cette valeur tant que la présence d'une contraction auriculaire gauche est avérée par les moyens d'analyse.

9. Le dispositif de la revendication 1, dans lequel le dispositif est essentiellement dépourvu de moyens de délivrance d'impulsions de stimulation ventriculaire.

10. Le dispositif de la revendication 1, dans lequel le dispositif est essentiellement dépourvu de moyens de recueil de dépolarisations ventriculaires.

## Patentansprüche

1. Aktive implantierbare medizinische Vorrichtung in Form einer Herzprothese zur Stimulierung, Resynchronisation und/oder Defibrillation, umfassend:
- Mittel (16) zur Erfassung von Depolarisationen des rechten Vorhofs;
- Mittel (18) zur Erfassung von Depolarisationen des linken Vorhofs;
- Mittel (18) zur Abgabe von Impulsen zur Stimulierung des linken Vorhofs;
- einen Kontraktionssensor (20), der in der Lage ist, ein Kontraktionssignal auszugeben, das repräsentativ für die durch die zyklischen Kontraktionen des Herzmuskels erzeugten Bewegungen ist;
- Mittel zur Analyse des Kontraktionssignals, die in der Lage sind, das Auftreten einer von der ventrikulären Kontraktion desselben Herzzyklus zeitlich unterscheidbaren tatsächlichen Kontraktion des linken Vorhofs festzustellen;
- Steuerungsmittel, die in der Lage sind, den Mitteln zur Abgabe von Impulsen zur Stimulierung des linken Vorhofs eine interaurikulare Verzögerung zwischen der Erfassung einer Depolarisation des rechten Vorhofs und der Abgabe von Impulsen zur Stimulierung des linken Vorhofs vorzuschreiben, wobei diese interaurikulare Verzögerung in Abhängigkeit des Kontraktionssignals veränderbar ist,
**dadurch gekennzeichnet, dass** die Steuerungsmittel Mittel sind, die in der Lage sind:
• wenn die Mittel zur Analyse des Kontraktionssignals die Abwesenheit einer Kontraktion des linken Vorhofs feststellen, im Laufe von aufeinanderfolgenden Herzzyklen die interaurikulare Verzögerung von einem Ausgangswert bis zu einem angepassten Wert iterativ zu reduzieren, der das Auftreten einer durch die Mittel zur Analyse des Kontraktionssignals festgestellten Kontraktion des linken Vorhofs gewährleistet, und
• den angepassten Wert der interaurikulären Verzögerung anschließend aufrechtzuerhalten, solange das Vorhandensein einer Kontraktion des linken Vorhofs durch die Mittel zur Analyse des Kontraktionssignals nachzuweisen ist,
um die diastolische Funktion des Patienten wiederherzustellen.

2. Vorrichtung nach Anspruch 1, wobei der Kontraktionssensor auf einer Sonde angeordnet ist, die an einem der Vorhöfe implantierbar ist.

3. Vorrichtung nach Anspruch 1, wobei der Kontraktionssensor auf einer Sonde angeordnet ist, die am rechten Vorhof implantierbar ist.

4. Vorrichtung nach Anspruch 1, wobei der Kontraktionssensor ein Sensor zur Bestimmung der endokardialen Beschleunigung ist.

5. Vorrichtung nach Anspruch 4, wobei die Analysemittel Mittel umfassen, die in der Lage sind, im durch den Sensor ausgegebenen Signal eine Komponente EA4 zu erkennen und zu isolieren, die der mit der Vorhof-Aktivität verbundenen vierten endokardialen Beschleunigungsspitze entspricht, wobei das Vorhandensein dieser Komponente EA4 das Auftreten einer Kontraktion des linken Vorhofs offenbart, die von der ventrikulären Kontraktion desselben Herzzyklus zeitlich unterscheidbar ist.

6. Vorrichtung nach Anspruch 1, wobei der Kontraktionssensor ein Sensor zur Bestimmung des linken endokavitären Blutdrucks ist.

7. Vorrichtung nach Anspruch 1, weiter umfassend Mittel zur Messung, bei einem spontanen Rhythmus ohne Vorhofstimulierung, eines Zeitabstands zwischen der Erfassung einer Depolarisation des rechten Vorhofs und der Erfassung einer darauffolgenden Depolarisation des linken Vorhofs, und zur Bestimmung des Ausgangswerts der interaurikularen Verzögerung anhand des gemessenen Zeitabstands.

8. Vorrichtung nach Anspruch 1, weiter umfassend Mittel, die in der Lage sind, in einem Fall, in dem der angepasste Wert unter einen vorgegebenen Mindestwert reduziert wird, ohne dass das Vorhandensein einer Kontraktion des linken Vorhofs durch die Analysemittel erfasst wird, die Abgabe von Impulsen zur Stimulierung des linken Vorhofs mit einem Kopplungsintervall zu steuern, das bis zu einem Wert iterativ reduziert wird, der das Auftreten der Kontraktion des linken Vorhofs gewährleistet, wobei dieser Wert des Kopplungsintervalls anschließend aufrechterhalten wird, solange das Vorhandensein einer Kontraktion des linken Vorhofs durch die Analysemittel festgestellt wird.

9. Vorrichtung nach Anspruch 1, wobei die Vorrichtung im Wesentlichen keine Mittel zur Abgabe von Impulsen zur Stimulation des Ventrikels aufweist.

10. Vorrichtung nach Anspruch 1, wobei die Vorrichtung im Wesentlichen keine Mittel zur Erfassung von ventrikularen Depolarisationen aufweist.

## Claims

1. An active implantable medical device of the cardiac prosthesis type for stimulation, resynchronisation and/or defibrillation, comprising:
- means (16) for capturing right atrial depolarisations;
- means (18) for capturing left atrial depolarisations;
- means (18) for delivering left atrial stimulation pulses;
- a contraction sensor (20) that is able to deliver a contraction signal that is representative of the movements produced by the cyclical contraction of the myocardium;
- means for analysis of the contraction signal that are able to reveal the occurrence of an effective left atrial contraction that is temporally distinguishable from the ventricular contraction of the same cardiac cycle;
- control means that are able to determine and apply an interatrial delay time between the capture of a right atrial depolarisation and the delivery of a left atrial stimulation pulse to the means for delivering left atrial stimulation pulses, wherein this interatrial delay time can be modified in accordance with the contraction signal,
**characterized in that** the control means are means that are able:
• in the absence of a left atrial contraction revealed by the means for analysis of the contraction signal, to iteratively reduce the interatrial delay time over consecutive cardiac cycles from an initial value to an adjusted value ensuring the occurrence of a left atrial contraction revealed by the means for analysis of the contraction signal, and
• to maintain the interatrial delay time's adjusted value as long as the presence of a left atrial contraction is revealed by the means for analysis of the contraction signal,
so as to restore the patient's diastolic function.

2. The device according to claim 1, wherein the contraction sensor is disposed on a probe that is implantable at one of the atriums.

3. The device according to claim 1, wherein the contraction sensor is disposed on a probe that is implantable at the right atrium.

4. The device according to claim 1, wherein the contraction sensor is an endocardial acceleration sensor.

5. The device according to claim 4, wherein the analysis means include means that are able to identify and isolate a component EA4 in the signal delivered by the sensor, corresponding to the fourth endocardial acceleration peak associated with the atrial activity, wherein the presence of this component EA4 reveals the occurrence of a left atrial contraction that is temporally distinguishable from the ventricular contraction of the same cardiac cycle.

6. The device according to claim 1, wherein the contraction sensor is a left endocavitary blood pressure sensor.

7. The device according to claim 1, further comprising means for measuring, in a spontaneous rhythm without atrial stimulation, the time interval separating the capture of a right atrial depolarisation and that of a consecutive left atrial depolarisation, and for determining said initial value of the interatrial delay time based on the measured time interval.

8. The device according to claim 1, further comprising means that are able, when the adjusted value is reduced below a minimal value without a left atrial contraction being detected by the analysis means, to control the delivery of left atrial stimulation pulses with a coupling interval that is iteratively reduced to a value ensuring the appearance of said left atrial contraction, wherein that value of the coupling interval is then maintained as long as the existence of a left atrial contraction is revealed by the analysis means.

9. The device according to claim 1, wherein the device substantially does not have means for delivering ventricular stimulation pulses.

10. The device according to claim 1, wherein the device substantially does not have means for capturing ventricular depolarisations.
